# EUROPEAN PATENT APPLICATION

(11) **EP 2 140 841 A1**
(43) Date of publication of application: **06.01.2010**
(21) Application number: 08012164.3
(22) Date of filing: 04.07.2008
(51) Int. Cl.: A61F 2/44

(54) **Device for closing spinal disc annulus**

(71) Applicant: Arthro Kinetics AG, 73728 Esslingen (DE)
(72) Inventor: Loveridge, Jason, Dr., 78240 Chambourcy (FR); Graeve, Thomas, Dr., 70597 Stuttgart (DE)
(74) Representative: Schwahn, Hartmut

(57) **Abstract**

The present invention relates to a device (100) and method for closing openings or apertures in a biological tissue, more particularly for closing an opening in a spinal disc annulus, for example, created during a diagnostic and/or therapeutic procedure, or an annular tear or disc herniation site.

## Description

### FIELD OF THE INVENTION

The present invention relates to a device and method for closing openings or apertures in a biological tissue, more particularly for closing an opening in a spinal disc annulus, for example, created during a diagnostic and/or therapeutic procedure, or an annular tear or disc herniation site.

### BACKGROUND

Intervertebral discs are located between adjacent vertebral bodies in the spine. They provide structural support for the spine and distribute forces exerted on the vertebral column. Intervertebral discs comprise three major components: cartilage endplate, nucleus pulposus, and annulus fibrosus. The central portion, i.e. nucleus pulposus, is relatively soft and gelatinous and contains about 70% to 90% water. Proteoglycan content is high and a significant amount of Type II collagen and cells are contained therein.

The annulus fibrosus surrounds the nucleus. It has a more rigid but elastic consistency and mainly comprises a fibrous network of 40% Type I collagen and 60% Type II collagen. Fibroblasts are distributed amongst the collagen scaffold. The annular portion mainly serves to provide peripheral mechanical support to the disc, afford torsional resistance, and contain the softer nuclear portion.

Under certain conditions the nucleus may extrude in part through an opening or tear in the annulus resulting from a mechanical overload of the spine. This condition is called disc herniation or hernia. The herniated matter may impinge on nerve roots in or near the spine causing pain or loss of sensation. The posterior and posterio-lateral portions of the annulus are most susceptible to attenuation or herniation. These portions are less resistant to the hydrostatic pressures of the nucleus.

Apertures in the spinal disc annulus may occur in connection with the treatment of herniation or can be created as a result of biopsy, for example, the surgical opening and/or removal of tissue from the spinal disk. Short-term and long-term structural and functional integrity of tissue is often an important consideration in arthroscopic surgery, especially in those situations where the apertures are slow to close and heal.

In the treatment of intervertebral disc disorders, in particular, disc herniation, a new or prosthetic nucleus can be placed within an intervertebral disc in place of the original nucleus pulposus which may eventually have left through the annulus tear. This is commonly accomplished by minimal invasive methods. For that, for example, a collapsed balloon mold prosthesis may be inserted through a cannula positioned through an opening within the annulus. The balloon may be filled with a flowable biomaterial that is adapted to provide a permanent disc replacement. Other methods of nucleus replacement foresee to place a folded strand orgel of biocompatible material via a tubular tool or cannula through the annulus opening.

There is a constant need to effectively seal the opening in the annulus which has either been initiated surgically, such as an access port for a minimally-invasive technique, or caused naturally, as in the case of herniation, after the nucleus replacement has been brought into place in particular or to prevent further prolapse of the nucleus pulposa. Various means to close the opening are known in the art. These means, however need complicated surgical procedures for effective application or are based on mechanically sophisticated solutions which are expensive and of less reliable function.

The technical problem underlying the present invention mainly pertains to the provision of a method, and means pertaining to that method, for a better and more safe closing of holes or apertures in tissues, in particular of the body of a human or non-human animal, more particular the sealing of an aperture through the annulus of a intervertebral disk.

A further object of the invention is to provide methods and means to permit the utilization of cheaper and more easy to provide materials for closing the opening or aperture.

The technical problem is solved by a method for sealing an aperture in an elastic tissue, in particular a biological tissue, more particular a cartilage tissue in situ, with a plug. The method comprising the steps of:
- expanding the aperture from its original diameter to a greater second diameter, preferably adapted to the dimensions of the plug, by stretching the cartilage tissue;
- inserting into the expanded aperture a plug that is preferably adapted to be sealably positioned within the aperture; and
- releasing the preferably elastically expanded aperture to return preferably to its original diameter to seal and hold the plug in place.

In short, the method of the invention is briefly characterized by the consecutive steps of expansion, insertion, and release of the aperture to be sealed.

According to this invention, a rather simply designed device or plug for sealing the aperture can be used, in particular without the need of further means or measures for expanding such a device to seal an aperture and to hold in place. The inventors have found that a plug that is inserted into an opening of the body according to the invention seals the opening or aperture tightly and safely and provides improved mechanical resistance. The stretching is mainly based on the elastic properties of the tissue that surrounds the aperture or opening.

In a preferred embodiment the opening is an aperture that is located in or extends through the annulus of an intervertebral disk. The inventors have found that the invention serves to safely seal the annulus and to prevent further hernia of the nucleus or escape of a prosthetic nucleus previously inserted into the central cavity of the intervertebral disk for replacing the nucleus pulposa.

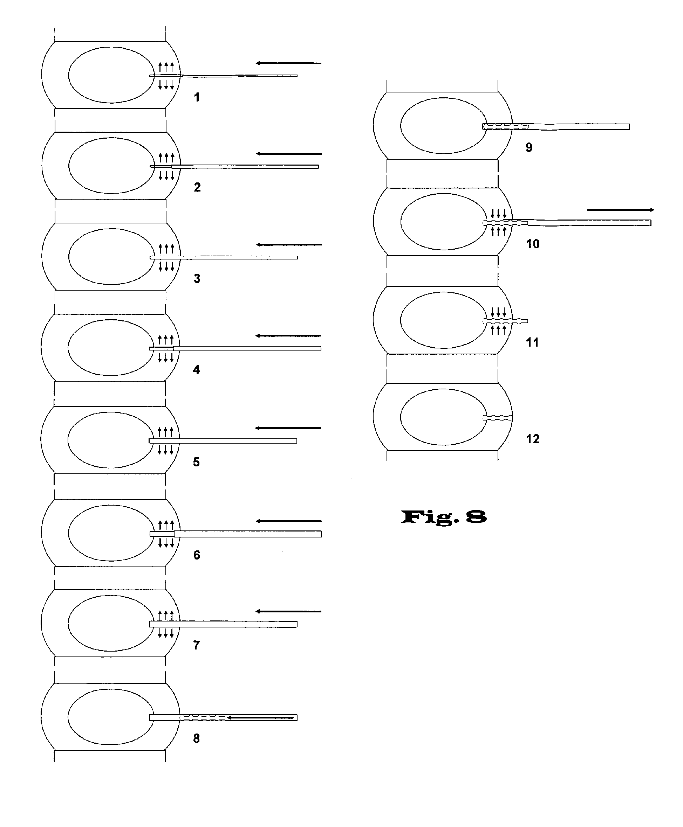

Figure 8 depicts a typical sequence for inserting a plug to seal an opening in the annulus. The opening or aperture is preferably located within an elastic tissue, in particular an elastic cartilage tissue or the like. In a first phase the aperture is expanded or stretched by external forces, in particular thus widening the aperture from its original diameter to a broader opening of greater diameter. Preferably due to the elasticity of the tissues the wall of the aperture is biased in the expanded or widened state. In that biased aperture an artificial device or plug is inserted to seal the aperture. The expansion of the aperture is then released with the tendency to return to its original smaller diameter. By that, the biased walls of the aperture tightly close around the inserted device or plug and support the sealing of the aperture and firmly hold it in place.

In a preferred embodiment, the expansion of the aperture is accomplished by consecutively inserting surgical instruments of preferably tubular form, in particular cannulae of increasing diameter.

The plug is preferably adapted to be used in combination with a biomaterial delivery cannula, in order to seal the cannula access aperture formed in the annulus in the course of delivering a biomaterial to the nucleus of the intervertebral disc. The cannulae are preferably of straight tubular form and preferably have a blunt and slightly tapered end. This design supports the stretching of the aperture and avoids abrasion of tissue material from the aperture.

More particular, the first cannula is adapted to fit the original diameter of the aperture and the last cannula in a row of consecutive cannulae of increasing diameter (outer diameter) is adapted to fit the dimensions of the plug. The last cannula yields the second diameter of the aperture for inserting the plug.

Preferably at that stage the plug is inserted into the expanded aperture, preferably through the cannula that is fitted to the dimensions of the plug in the inside and to the diameter of the expanded annulus on the outside. Within the cannula the plug is moved preferably with a plunger and brought into the final position, i.e. matching the position of the aperture's walls.

For example, after the aperture is widened a nucleus implant or prosthesis may be introduced through the surgical tools into the nucleus and, after that, the device may be placed in position, preferably via the same tool.

The plug is preferably positioned to span the aperture in longitudinal direction to the most possible extend. Preferably, care is taken not to insert the plug too deep into the nucleus. Without wishing to be bound to the theory, if the plug is advanced too far into the central part of the disk, the intra-nucleus pressure increases under mechanical stress and the plug may be discharged from the aperture. Once the plug is in its final position for sealing the aperture, this cannula is retracted and the biased walls of the expanded aperture are released and "collapse" onto the inserted plug.

The second concept of the invention relates to a plug or device for sealing an aperture or opening in a preferably elastic tissue, in particular cartilage tissue. The plug serves to seal the aperture in situ. The plug is adapted to be positioned within the aperture and to seal the aperture preferably only by elastic tension of the preferably elastic tissue that surrounds the aperture, for example, the collagen scaffold in the annulus of an intervertebral disc. The elastic tension forces are primarily directed perpendicular to the surface of the plug that is mating with the aperture's wall.

In contrast to known devices for sealing apertures in the body, the invented plug is adapted not to expand within the aperture. It is specifically designed to withstand compressive forces exerted by the walls of the aperture that are biased through previous expansion according to the first concept of the invention.

In a preferred embodiment, the plug is constituted of a material that is less compressible than the elastic tissue, in particular a cartilage tissue that surrounds the opening or aperture. More preferred, the plug is constituted of a material that is basically non-elastic and non-deformable. It goes without saying that the plug is flexible enough to be applied to the aperture through a cannula as described herein and is non-deformable relative to the surrounding tissue that holds the plug in place.

In a preferred embodiment, the plug is constituted of a material that is basically non-porous. In connection with the invention the term "non-porous" relates to surfaces having no pores relative to the size of tissue cells, i.e. surfaces comprising solely pores that are smaller than the cells and where the cells cannot grow in or migrate in are considered "non-porous" in the terms of the invention. In particular, surfaces with pores having diameter of 1 µm or less, more preferred 500 nm or less, are considered "non-porous". According to the invention there is no need for cell material to grow into the plug from the surrounding tissue. Nevertheless, the plug material may be absorbable for being absorbed or dissolved fully or in part over time.

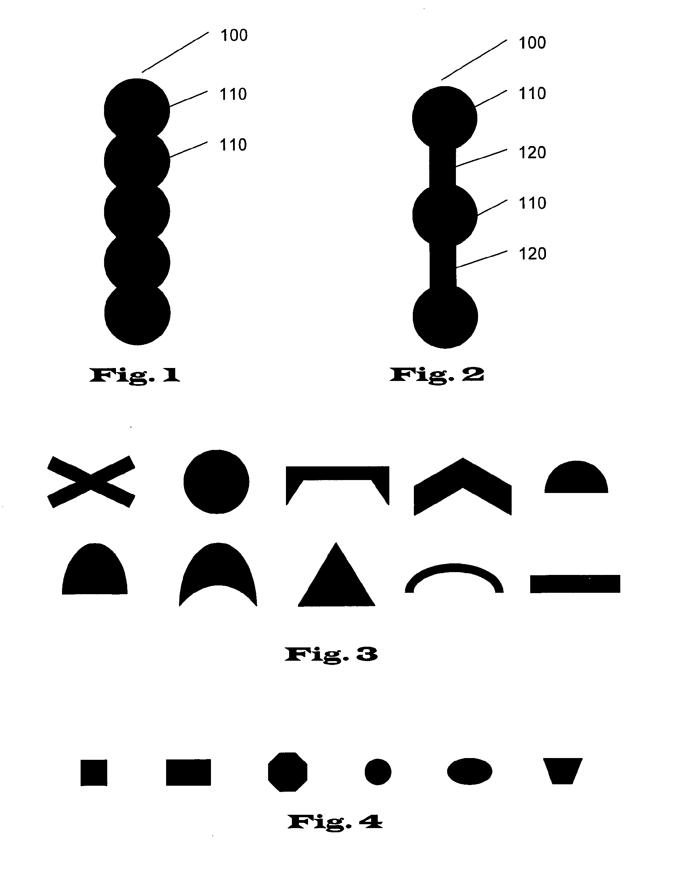

In a preferred embodiment, the plug is provided in configurations selected from the group consisting of cylindrical plugs, tubular forms, and elongated, curved forms. Details on the cross-sections of the plug are set forth in Figures 3 and 4. Along the longitudinal axis of the plug, i.e. the axis spanning or extending through the aperture to be sealed, the plug may exhibit indentations, grooves, protrusions, or recessions. Such structures may support fixation of the device within the aperture and/or sealing. These structures, however, in the meaning of the invention do not represent the sole reason or means for fixation. The main fixation effect is conferred through friction between the outer surface of the plug and the biased walls of the aperture that collapse over the plug and, by that, hold it tightly and firmly in place.

In an alternative embodiment the elastic modulus of the plug material is specifically adapted to and preferably matched with the elastic modulus of the tissue that surrounds the aperture. This reduces mechanical shear forces between the plug and the walls of the aperture to be sealed that may occur during mechanical loading thus preventing escape of the plug from the aperture and supporting the sealing.

Another aspect of the invention is the use of said device or plug for sealing an opening or aperture in an elastic tissue of the body, in particular a cartilage tissue.

The invention further concerns a method for treating a cartilage defect, in particular a hernia of the intervertebral disk, in a human or non-human animal the method comprising at least the steps of the method of sealing an opening as described herein.

The invention further concerns a surgical kit comprising at least the device in combination with a surgical tool adapted to insert and position the device within an aperture in a cartilage tissue to seal the aperture.

The invention will be further illustrated in the accompanying drawing and description as follows:
Fig.1 and 2 are overall schematic depictions of a plan view of the device of plug to be used to close the aperture.
Fig. 3 and 4 are schematic depictions of transverse sections of the device according to Figs. 1 or 2.
Fig. 5, 6 and 7 are overall schematic depictions of a portion of the spine wherein the device is positioned within an annular aperture in the intervertebral disc; Figs. 5 and 7: top view, Fig. 6: side view.
Fig. 8 is am schematic depiction of the operation sequence for installing the plug into an aperture on the annulus to seal it.
Fig. 9 is a top view on a intervertebral disk with a nucleus prosthesis and a sealing device that has been installed according to the invention.

As will be apparent from the drawings, the present invention concerns an implant, device or plug for closing openings in a primarily elastic biological tissue and in particular for closing openings in the annulus fibrosus to prevent the expunging of the nucleus pulposa or a prosthetic nucleus from the spinal disc. The plug can be placed in any suitable primarily elastic tissue site containing an aperture which would benefit from safe sealing for restoration of physiological function, healthy conditions, or for healing.

Typically, such apertures are produced as a result of injury, disc hernation, or created surgically, such as at biopsy sites, as access apertures for surgical instruments, for example, catheter or cannulae, or insertion of an implant, for example, a prosthetic nucleus. Non-vascularised tissue sites, such as the annulus of the intervertebral disc, are typically more difficult to heal. It has been shown before that non-vascularised tissue sites, particularly those prone to hernation, tend to benefit from immediate and secure closure and are particularly suited for the invention.

Without wishing to be bound to the theory, the elastically biased surrounding of the aperture creates frictional forces between the aperture wall and the surface of the plug. This advantageously serves to hold the plug in place and prevents the escape of the plug from the aperture, and in particular of the nucleus pulposa and/or the implanted prosthetic nucleus, through the aperture. In a preferred embodiment the shape and nature of the surface of the plug support the friction between the plug and the elastically biased surrounding of the aperture. Additional anchoring effects are preferably accomplished by providing indentations, grooves, protrusions, or recessions on the surface of the plug.

A further advantage of this plug over those known in the art is that it does not need to be screwed into place. Neither does it require component parts, which are in contact with the tissue and primarily hold the device in place, to be retracted for insertion or placement into the hole in the tissue nor extended or expanded to retain the device in the hole in the tissue. The device relies simply on friction between itself and the surrounding tissue to stay in place and therefore is simpler and easier to position in the correct location. Furthermore, because this novel plug does not rely on extendable or expandable elements to hold it in place it is easier to remove if the device is found to be incorrectly placed or where placement results in other unwanted effects, such as pain, for example, or where the device needs to be removed to replace an older device due to wear or to insert a larger device if the hole grows larger.

In the case of the spinal disc, it is not obvious that such a simple device would be sufficient to prevent the nucleus pulposa from forcing the device out of the hole it is filling in the annulus fibrosus as a result of the large pressure exerted by the weight of the body and the movement on the spine.

In preferred embodiments the plug mainly extends in the direction of the tubular aperture and spans through the tissue. Such a plug is preferably mainly of a "bi-directional" shape and exhibits symmetry on the axis of its primary extension (longitudinal axis). It can be introduced or placed in the aperture in either direction or orientation. In another preferred embodiment the plug exhibits a thickening on one side and is optimally placed in one direction, preferably with the thickened part facing towards the inside of the aperture, i.e., for example, towards the nucleus of the intervertebral disk.

The device can be manufactured from any biocompatible material including for example; metals such as titanium, alloys, ceramics, composite materials, glass, hydrogels or other natural or synthetic polymers. In more preferred embodiments, the plug or device comprises or consists of materials selected from polyamides polyethylenes (LDPE, HDPE, UHMWPE, PET), polyetherketones (PEEK), polyacryletherketones (PEKK), polymethacrylates (PHEMA, PMMA), polyfluoroethylenes (PTFE), polyurethanes (PU), silicones and silicone-elastomers (SI) and mixtures or co-polmers thereof. The invention is not limited to the above materials which stand as examples. Other bio-compatible materials are also included with the invention.

In preferred embodiments the materials are specifically designed to be either non-resorbable and chemically resistant or, in a preferred version, to be resorbable over a certain period of time, for example, after a transplanted nucleous prosthesis has been successfully grown in the nuclear part of the intervertebral disk. The annulus tissue will close over the aperture as the device is resorbed.

The surface of the device is preferably rough and/or can be or is coated with a biocompatible material which may contain bio-active ingredient such as a drug or enzyme.

In a preferred embodiment the device or plug comprises materials that are radio-opaque, i.e. can be seen by non-invasive means, for example X-ray or MRI. This may serve to ensure correct positioning of the device in place during surgery.

In general, the device or plug is designed or trimmed to fit onto the aperture or opening. For example, the device may measure up to 20 mm in diameter and 40 mm in length, but may also be as small as 1 mm by 1 mm. The device will be adapted to the dimensions of the aperture, taking into account the stretching of the aperture according the preset invention. For example, the diameter of the device is of about 5% or more, preferably 20% or more, more preferred 50% or more larger diameter than the aperture's opening. According to the invention, the device or plug is always oversized in comparison to the dimensions of the aperture to be sealed.

In preferred variants, in particular for the closing of apertures in the annulus of a intervertebral disk, the length (longitudinal extension) can measure from 5 to 20 mm; its width (diameter) can measure from 2 to 10 mm. It goes without saying that the exact dimensions and shape of the plug are selected depending on the size and nature of the hole or aperture in the tissue to be filled and sealed. The plug is preferably fitted in such a way as to completely fill the aperture in the tissue.

Preferred embodiments of the device are depicted in Figures 1 to 4.
Figure 1 shows a device or plug (100) comprising at least one of or a plurality of projections (110). Projections of that kind may be arranged in series and in the simplest case are formed by a series of spheres of preferably equal diameter. Figure 2 depicts an alternative variety of the device of Figure 1 comprising at least one projecting element (110) and a stem core section (120) connecting the projecting element (110). In Figure 2 a device (100) having, for example, two projective elements (110) at each end and a third projective element (110) arranged, for example, in about equal distance between both tail elements. In this case two intercalating or bridging elements (120) are arranged between the three projecting elements (110). In the preferred embodiment of Figure 2 the projecting elements (110) are in the form of a sphere; the bridging elements (120) are preferably of cylindrical shape.

Figures 3 and 4 schematically depict cross-sections of preferred structured projections (110) and bridging elements (120), respectively. Devices comprising any combination of structures according Figure 3 and according Figure 4 form part of the present invention.

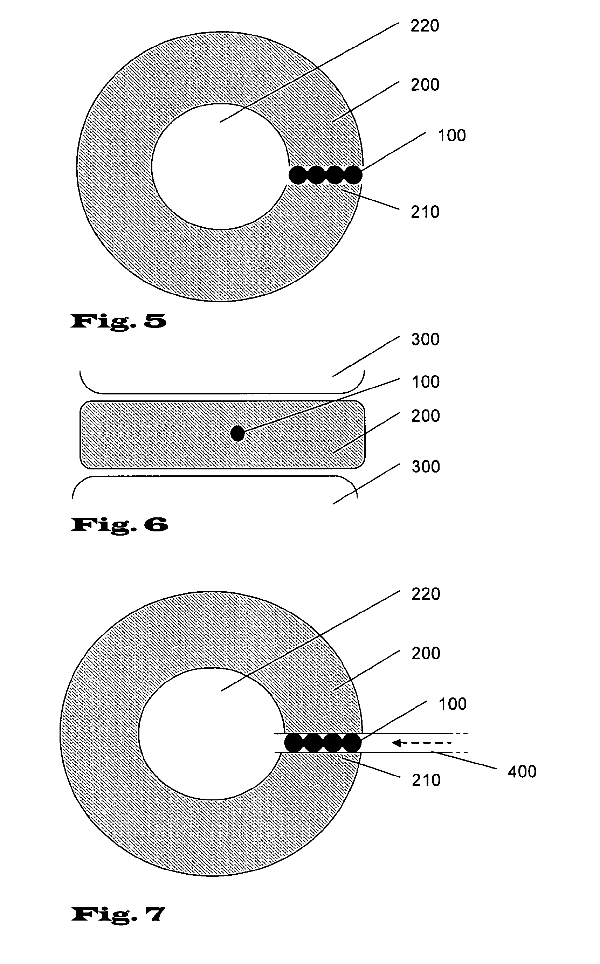

Turning now to Figures 5 to 7, in the method of installing the device and sealing the aperture the device (100) is arranged through an aperture (210) or through an annulus (200) of an intervertebral disk. The device (100) serves to seal the volume, for example, of the inner nucleus or central cavity (220) of an intervertebral disk and to prevent escape of material from the nucleus (220). The walls of the aperture (210) are in intimate contact with the surface of the device (100). The indentations and protections (110) provided by the device (100) are essentially dovetailing with the surrounding tissue. Figure 7 schematically depicts the basic principle of the instalment of the device (100) in the aperture (210) according to the invention. For example, a surgical tool (400), preferably a wire probe or tubular cannula is inserted in the aperture (210) thereby stretching the walls of the aperture (210) and widening the opening. The device (100) is brought in place through the inside of the tubular tool (200). The preferably tubular tool (400) is retractable thus yielding an intimate contact of the walls of the aperture (210) with the device (100), preferably through previous pre-stretching of the walls.

Turning now to Figure 8, a typical surgical procedure for sealing an opening in a tissue, for example, a tear in the annulus of an intervertebral disk, is depicted. Figure 8 depicts a series of consecutive steps 1 to 12. Further intermittent steps may be added to the procedure without leaving the scope of the present invention. The insertion of the plug or device into the tissue may be aided by a tube, needle or sleeve, for example, inserted into the hole in the tissue to place the device and then withdrawn to leave the device in place in the aperture as shown in the cartoon:

In steps 1 to 7 cannulae of increasing diameter are introduced into the aperture for the purpose of widening the aperture. In preferred embodiments, that are adapted for use to close an annulus aperture, the first tool to be inserted is a guiding wire of 1 mm diameter, followed by a first cannula of 2 mm, a second cannula of 3.5 mm, and a third cannula or 4 mm, each cannula having rounded or tapered ends to facilitate insertion. The proceeding, thus smaller cannula serves as a guide for the consecutive cannula, which is preferably simply shipped over the cannula of smaller diameter. Preferably the outer diameter of proceeding cannula or wire and inner diameter of consecutive cannula are essentially matched.

Through the last cannula, which is adapted to the dimensions of the device, for example, with an outer diameter of 4 mm and an inner diameter of 3.5 mm, the device is installed within the aperture (step 8, details in step 8a) once the device is brought into place (step 9), the hollow cannula is retracted (step 10), leaving the device in place (step 11). Optionally, the protecting part of the device can be trimmed to yield a smooth annulus surface (step 12).

### 1. Preparation

### Examples sealing annulus apertures in a calf model

Twelve lumbal monosegmental vertebra preparations from the calf: 4T13-L1, 4L2-L3 and 4L4-L5 Segments were obtained from the slaughterhouse. For storage, the preparations were tightly sealed in plastic bags and kept at approximately -28° C. Preparation took place at about 4° C. While preserving the tendons, articular capsules and intervertebral disks the remaining soft tissue (muscle, connective tissue, fat, etc.) was removed. The caudal and cranial ends of the vertebral body were both imbedded in resin (Polymethylmetacrylat, PMMA, e.g. Technovit-3040), with care taken to arrange the intervertebral bodies in the preparations strictly horizontal. For installing the preparations in an apparatus for mechanical testing, flanges were attached to the resin blocks at each end of the preparations.

### 2. Sealing the annulus aperture

For installing the sealing device the annulus was first punctured with a solid probe wire of 1 mm diameter. The probe was left in place and served as a guide for the consecutive tool, a first tubular cannula of 2 mm outer diameter. The probe wire was removed and the first cannula served as a guide for a second cannula of 3.5 mm outer diameter that was introduced into the annular opening, with care taken not to disrupt or ablate annulus tissue with the tool. The first cannula was removed and the second cannula served as a guide for a third cannula of 4 mm outer diameter. By that, the annulus opening was expanded to approximately 4 mm diameter.

Through the last 4 mm cannula surgical instruments were introduced into the nucleus to cut and remove nucleus matter. Through the same cannula a prosthetic nucleus, adapted to the inner diameter of cannula and to the volume of the central cavity, was introduced into the nucleus by means of a plunger. After the nuclear prosthesis was installed the plunger was removed and the plugging device of 3 to 5 mm diameter was introduced through the same cannulae which was left in place after insertion of the nuclear prosthesis. With the aid of a plunger the device was brought into position in the annulus wall. By means of the plunger the plug device was held in place while the cannula was retracted from the annulus aperture. The pre-stretched tissue of the annulus collapsed over the device. Eventually, remainder parts of the plug that projected from the annulus ring was cut away leaving a smooth surface on the outer part of the annulus.

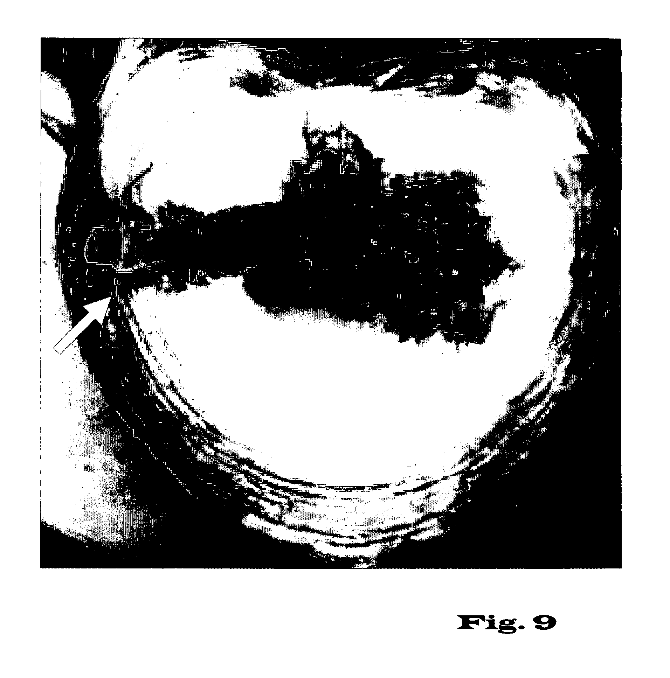

In figure 9 an intervertebral disk is depicted with the plug installed in place (left side, arrow).

### 3. Mechanical testing

The preparations were installed into a standardized mechanical simulator for periodically stressing the vertebra preparations. The mechanical stress experiments were performed at room temperature while the preparations were constantly wetted by physiological saline.

## Claims

1. A method for sealing an aperture in a cartilage tissue in situ with a plug, comprising the steps of:
- expanding the aperture from its original first diameter to a greater second diameter, adapted to the dimensions of the plug, by elastically stretching the cartilage tissue;
- inserting into the expanded aperture a plug adapted to be positioned within the aperture; and
- releasing the expanded aperture for returning to the original first diameter to seal and hold the plug in place.

2. The method of claim 1, wherein the expansion of the aperture is accomplished by consecutively inserting cannulae or probes of increasing diameter.

3. The method of claim 2, wherein the first cannula or probe is adapted to fit the original first diameter and the last cannula is adapted to fit the dimensions of the plug, yielding the second diameter of the aperture.

4. The method of claim 2 or 3, wherein the plug is inserted to the aperture through the cannula that is fitted to the dimensions of the plug.

5. The method of any one of the preceding claims, wherein the aperture is in the annulus of an intervertebral disc.

6. The method of any one of the preceding claims, wherein the plug is adapted to be used in combination with a biomaterial delivery cannula, in order to seal the cannula access aperture formed in the annulus in the course of delivering a biomaterial to the nucleus of the intervertebral disc.

7. A plug for sealing an aperture in a cartilage tissue in situ, the plug being adapted to be positioned within the aperture only by elastic tension of the cartilage tissue surrounding the aperture.

8. The plug of claim 7, the plug being adapted not to expand inside the aperture.

9. The plug of claim 7 or 8, the plug being constituted of material that is adapted to the elastic modulus of the biological tissue surrounding the aperture and the plug.

10. The plug of any one of claims 7 to 9, the plug being constituted of material that is non-porous.

11. The plug of any one of claims 7 to 10, the plug being provided in a configuration selected from the group consisting of cylindrical plugs, tubular forms, and elongated, curved forms.

12. Use of a device according to any one of claims 7 to 11 for sealing an aperture in a cartilage tissue.

13. A method of treating a cartilage defect in a human or non-human animal comprising the steps of the method of any one of claims 1 to 6.

14. A surgical kit comprising the plug according to any one of claims 7 to 11 in combination with at least one surgical tool, cannula or probe, adapted for insertion and positioning of the plug within an aperture in a biological tissue to seal the aperture.

15. The surgical kit of claim 14, **characterized in that** the surgical tools comprise two or more tubular cannulae of increasing diameter.

## Amended claims

### Amended claims in accordance with Rule 137(2) EPC.

**1.** A plug for use in a method of treatment of a cartilage defect in a human or animal body by sealing an aperture in a cartilage tissue in situ with the plug to be positioned within the aperture, **characterized in that** the plug is not expandable inside the aperture and **in that** the plug is oversized in comparison to the dimensions of the aperture.

**2.** The plug according to claim 1, wherein the method for sealing an aperture in a cartilage tissue in situ with a plug, comprises the steps of:
- expanding the aperture from its original first diameter to a greater second diameter, adapted to the dimensions of the plug, by elastically stretching the cartilage tissue;
- inserting into the expanded aperture a plug adapted to be positioned within the aperture; and
- releasing the expanded aperture for returning to the original first diameter to seal and hold the plug in place.

**3.** The plug according to claim 2, wherein in the method for sealing an aperture the expansion of the aperture is accomplished by consecutively inserting cannulae or probes of increasing diameter.

**4.** The plug according to claim 3, wherein in the method for sealing an aperture the first cannula or probe is adapted to fit the original first diameter and the last cannula is adapted to fit the dimensions of the plug, yielding the second diameter of the aperture.

**5.** The plug according to claim 3 or 4, wherein in the method for sealing an aperture the plug is inserted to the aperture through the cannula that is fitted to the dimensions of the plug.

**6.** The plug according to any one of the preceding claims, wherein the aperture is in the annulus of an intervertebral disc.

**7.** The plug according to any one of the preceding claims, wherein the plug is adapted to be used in combination with a biomaterial delivery cannula, in order to seal the cannula access aperture formed in the annulus in the course of delivering a biomaterial to the nucleus of the intervertebral disc.

**8.** The plug according to any one of the preceding claims, being constituted of material that is matched with the elastic modulus of the biological tissue surrounding the aperture and the plug.

**9.** The plug according to any one of the preceding claims, being constituted of material that is non-porous.

**10.** The plug according to any one of the preceding claims, being in a configuration selected from the group consisting of cylindrical plugs, tubular forms, and elongated, curved forms.

**11.** A surgical kit for sealing an aperture in a cartilage tissue in situ comprising:
- a plug for sealing the aperture in a cartilage tissue by being positioned within the aperture;
- means for expanding the aperture from its original first diameter to a greater second diameter, adapted to the dimensions of the plug, by elastically stretching the cartilage tissue;
- means for inserting the plug into the expanded aperture; and
- means for releasing the expanded aperture for returning to the original first diameter to seal and hold the plug in place.

**12.** A surgical kit according to claim 11, wherein the means for expanding the aperture, the means for inserting the plug into the expanded aperture, and the means for releasing the expanded aperture are selected from one or more of tubular cannulae of increasing diameter.
